# EUROPEAN PATENT APPLICATION

(11) **EP 3 518 248 A1**
(43) Date of publication of application: **31.07.2019**
(21) Application number: 18153928.9
(22) Date of filing: 29.01.2018
(51) Int. Cl.: G16H 50/50

(54) **PROVIDING SUBJECT-SPECIFIC INFORMATION**

(71) Applicant: Koninklijke Philips N.V., 5656 AE Eindhoven (NL)
(72) Inventor: ZHU, Qin, 5656 AE Eindhoven (NL); CHAN, Tak Ming, 5656 AE Eindhoven (NL); JIANG, Jie, 5656 AE Eindhoven (NL); HUO, Yong, 5656 AE Eindhoven (NL)
(74) Representative: de Haan, Poul Erik

(57) **Abstract**

Presented are concepts for providing subject-specific information. One such concept comprises obtaining a first knowledge model, the first knowledge model comprising a plurality of clinical data elements for a population of subjects and relationship information representing one or more relationships between the clinical data elements. Also obtained is historical data relating to a sub-population of the population of subjects, the historical data relating to previously-obtained clinical data elements and relationship information for the sub-population. A second knowledge model is generated based on the first knowledge model and the historical data. A subject-specific model is generated based on the second knowledge model and subject-specific data relating to a subject.

## Description

### FIELD OF THE INVENTION

This invention relates to the field of subject information provision and more particularly to the provision of subject-specific information.

### BACKGROUND OF THE INVENTION

Known systems for providing subject information (such as clinical data, patient data and the like) are typically arranged to provide (e.g. display) a list of data elements, such as a list of values for clinical or medical parameters.

A conventional approach to providing such a list includes ordering the data elements alphabetically. When the clinical data elements are ordered alphabetically, a user (e.g. doctor, clinician, technician, medical professional, etc.) may be provided with a list of items from which it may be required to find a desired or relevant data element.

For a subject having numerous medical events (e.g. a patient suffering from chronic diseases and/or regularly visiting a medical practitioner), clinical information relating to the subject may be extensive. Exploration and/or analysis of the subject's clinical information may therefore be resource intensive and/or difficult.

With intuitive presentation and navigation of subject-specific clinical information being of paramount importance in Clinical Decision Support (CDS), there remains a need for an approach to providing subject-specific information in a manner which is simple, intuitive and supports identification of potential relationships between data elements.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for providing subject-specific information, the method comprising: obtaining a first knowledge model, the first knowledge model comprising a plurality of clinical data elements for a population of subjects and relationship information representing one or more relationships between the clinical data elements; obtaining historical data relating to a sub-population of the population of subjects, the historical data relating to previously-obtained clinical data elements and relationship information for the sub-population; generating a second knowledge model based on the first knowledge model and the historical data; obtaining subject-specific data relating to a subject, the subject-specific data comprising a plurality of clinical data elements for the subject; and generating a subject-specific model based on the second knowledge model and the subject-specific data.

Proposed is a concept of combining broad knowledge (e.g. a set of clinical knowledge for a broad population of subjects), a data analytics model (e.g. historical data relating to a subset of the broad population), and subject-specific knowledge (e.g. clinical data for the subject provided by a medical professional or physician) so as to generate subject specific information, which may facilitate the discovery of associated clinical events accurately and efficiently for example.

For example, proposed concepts may obtain a generic knowledge model comprising a simple list of clinical events and their relationships for a large, broad set of subjects, and this may be based on existing/general clinical knowledge for example. Such a generic knowledge model may, for instance, relate to a general population (and not to a specific individual) and thus be large in size and/or slow to update. Embodiments may then use historical data relating to a relevant sub-set of the large set (e.g. a subset of the general population) and to refine (e.g. 'train' or improve) the generic knowledge and thus generate a second knowledge model (which may be thought of as a data analytics model, for example). Such a second (refined) knowledge model may complement information or knowledge which is not normally available from the generic knowledge model alone. Put another way, a new (i.e. second) knowledge model may be generated having a sub-set of clinical events with more accurate information. Finally, the second (refined) knowledge model may be combined with subject-specific data relating to a subject (such as information from a physician's records relating to the subject's clinical history). In doing so, feedback information may be used to refine, confirm or otherwise improve the accuracy of the information and generate a subject-specific model. Such combination of subject-specific information (e.g. expert data from a medical professional and/or historical clinical data for the subject) thus enables the generation of a personalised model for a specific subject. Such a subject-specific model may provide much more accurate and/or concise data (e.g. with adjusted and/or specific relationships between clinical elements for the specific subject).

Embodiments may be formulated as a special sub-graph generation problem where a data analytics model extends a shared, broad graph of clinical knowledge. Such a resultant sub-graph may be used to extend a clinician's event feedback history for example.

Embodiments may thus propose the refinement of clinical data according to a specific subject (e.g. patient, individual or user), thereby facilitating the provision of subject-specific information in a manner which may be simple, intuitive and support identification of potential relationships between data elements. Thus, there may be provided concepts for assisting the presentation and/or navigation of subject-specific clinical information that enable improved CDS.

There may be proposed an approach to providing subject-specific information by combining general clinical knowledge, a data analytics/mining model, and expert (e.g. physician, medical professional, or the like) information to generate subject-specific clinical information that helps associated clinical events be identified accurately and/or efficiently.

Provision of subject-specific information by combining general clinical knowledge with historical information and subject-specific expert data may facilitate the provision and/or display of clinical information that can be combined with the graphical representation for simplified exploration and/or analysis. When working with such subject-specific clinical information and the graphical representation, clinical users may require less mental effort in identifying events and/or relationships.

Combination of clinical knowledge for a general population with data analytics for a sub-set of the population and subject-specific data may help a user to explore and/or analyse the subject-specific clinical data in a simple and efficient manner. For example, a reduced amount of data, more concise data, more relevant data, and/or more accurate data may be provided to a user for exploration.

Proposed concepts may thus increase the clinical value of information by enabling subject-specific clinical information to be provided in a manner which accounts for historical knowledge and/or expertise. This may support assessment, enable improved focus on a diagnosis, and/or support clinical planning. Improved CDS may therefore be facilitated by proposed concepts. Also, proposed embodiments may support visual navigation or exploration of patient-specific clinical (e.g. diagnostic) information.

By way of example, clinical events may be defined based on clinical data recorded by subjects. Based on categories, it may include (but not be limited to) the following examples: medication adherence related events, e.g. a patient stops to take one medication for two days; vital signs, e.g. a patient's average heart rate is larger than 140 but smaller than 160 3 days in last 5 days; health notes and symptoms, e.g. a patient recorded a dizzy symptom in recent 7 days; exercise, sleeping and nutrition recording, e.g. a patient sleeps less than 4 hours in one day.

Proposed embodiments may provide a set of clinical information comprising health domain content, clinical events and/or relationships between clinical data that are specific to a subject (such as a medical patient for example). Such clinical information may be generated with reference to a visualization protocol that provides an integrated and organized view of associated events combined with the clinical knowledge, data analytics model, and subject-specific data. It may also provide a way for a user to confirm and revise the subject's associated clinical events, which can then be stored as historical data for future use.

The first knowledge model may comprise clinical knowledge for a population of subjects (i.e. not an individual subject). For instance, a first knowledge model may relate to knowledge for patients in China. By way of example, it may be based on existing knowledge, rules, and professional experience. The first knowledge model may thus be a generic model with clinical events and their relationship weights. Due to a lack of specific clinical knowledge, the weights may be simplified into categories.

Historical data relating to a sub-population of the population of subjects can provide associated events for the sub-population (but not only a current subject (e.g. individual patient) of interest), and thus enable data analytics for use with the first knowledge model. By way of example, such historical data may relate to a sub-population of patients in Beijing with a particular disease.

Subject-specific data relating to an individual subject of interest may comprise feedback history for the subject, and so may relate to personalized clinical events. In this way, it may lack information regarding related events from the first knowledge model (e.g. general clinical knowledge) and/or the data analytics provide by the historical data relating to a sub-population.

Proposed embodiments may combine the various information and so may be viewed as combining various models of differing levels of abstraction/generality. For example, data analytics models using the historical data for a sub-population may first be used to adjust the first knowledge model. This may provide a second knowledge model relating to a sub-set of clinical events and having more accurate weights (e.g. continuous real numbers). The Subject-specific data (e.g. a physician's feedback history for a specific subject) can then be used to refine or adjust the second knowledge model in a similar manner. Consequently, there may be provided a subject-specific model relating to a sub-set of clinical events with subject-specific weightings.

In an embodiment, the step of generating a second knowledge model may comprise: based on the historical data relating to a sub-population of the population of subjects, processing clinical data elements of the first knowledge model for the sub-population to generate modified clinical data elements for the sub-population; based on the historical data relating to a sub-population of the population of subjects, processing relationship information representing one or more relationships between the clinical data elements of the first knowledge model for the sub-population to generate modified relationship information representing one or more relationships between the modified clinical data elements; and generating a second knowledge model comprising: the modified clinical data elements for the sub-population; and the modified relationship information representing one or more relationships between the modified clinical data elements.

By way of example, the following two events may be considered: Event 1 = a higher blood pressure defined as "Systolic Blood Pressure is greater than 180"; and Event 2 = a headache. In the first knowledge model, there may be a relationship between Event 1 and Event 2 if a time difference between their occurrences is less than one hour, and the relationship strength may be defined as 0.5. In a sub-population, e.g. patients with a diagnosis of hypertension, a strong correlation may be found between Event 1 and Event 2. Based on frequency sets, 75% of the data items may follow this pattern and so the relationship strength may be adjusted to 0.75. The correlation might be found, for example, from correlation analysis or regression analysis.

The subject-specific model may comprise a plurality of clinical data elements for the subject and subject-specific relationship information representing relationships between the clinical data elements for the subject. Thus, by way of example, a physician's historical feedback and confirmation data of clinical events for the specific patient may be constructed as a personalized model representing associated clinical events. A personalised model for a specific subject (e.g. patient) may thus be generated which assists in identification of clinical events and/or relationships unique to the subject.

In an embodiment, the step of generating a subject-specific model may comprise: based on the subject-specific data, modifying the second knowledge model to generate a subject-specific model.

For instance, the same example as given above may be considered, wherein Event 1 = a higher blood pressure defined as "Systolic Blood Pressure is greater than 180"; and Event 2 = a headache. In the second knowledge model, patients with a diagnosis of hypertension, the relationship strength between Event 1 and Event 2 is 0.75. However, for a specific subject of interest there may be recorded three instance/occurrences of Event 2, and no instance/occurrence of Event 1 happened within the valid time scope of Event 2. In such a case, for that specific patient of interest, the relationship strength between Event 1 and Event 2 may be decreased based on pre-defined rules (e.g. to 0.3), since it may be inferred that there is a reduced correlation between Event 1 and Event 2 for the specific subject of interest. A physician's confirmation of the relationship may help to improve the relationship strength accuracy (as well based on pre-defined rules), by taking account of professional feedback/input that is based on subject-specific observations.

By way of example, the relationship information representing one or more relationships between the data elements may comprise a weighting value representative of an association between two data elements. A list of clinical events may therefore be represented as a graph, wherein the weights of the edges are used to indicate different relationship strengths. Embodiments may therefore support simple and quick understanding of clinical events and/or relationships between the clinical events.

In an embodiment, at least one characteristic value of the subject and the sub-population may be the same. In this way, the second knowledge model may comprise information that is relevant to the subject, because the subject may have one or more characteristics in common with the sub-population. By way of example, the subject may be a member of the sub-population (e.g. the historical data may relate to a sub-population of patients in Beijing with disease A, and the subject may be a patient from Beijing with disease A). This may help to ensure that second knowledge model provides a complementary perspective of clinical events and their relationship(s) that may be relevant to the subject. This may be especially relevant when subject-specific data does not capture more general or broader clinical events and/or relationships.

In some embodiments, the step of obtaining subject-specific data relating to a subject may comprise: obtaining historical data comprising previously-generated clinical data elements for the subject. By way of example, obtaining previously-generated clinical data elements for the subject may involve receiving information from a physician or medical professional, wherein the information comprises feedback and/or confirmatory data relating to clinical events for the specific subject. This may enable a model to be constructed on a personalized basis, thus further refining or improving previously obtained model (such as the second knowledge model for example).

Proposed embodiments support the provision, presentation and/or navigation of subject-specific information in an improved (e.g. simple and more accurate) manner. Simple and intuitive presentation or navigation of subject-specific clinical information may thus be facilitated by proposed embodiments.

Proposed embodiments may enable a user to quickly and intuitively obtain subject-specific clinical information which may, for example, then be displayed to the user when creating a structured report. By using an input interface, relevant clinical information may be automatically provided, thereby avoiding time scrolling down long lists of clinical information and/or the mental effort in having to search or digest excessive information that might (or might not) be relevant. Embodiments may therefore facilitate the provision of clinical information in a manner which is tailored to a specific subject. This may, in turn, facilitate the provision of clinical information that is more intuitive and/or convenient for a user to explore or analyse.

According to another aspect of the invention, there is provided a method for displaying subject-specific information, the method comprising: providing subject-specific information according to any preceding claim; generating a display control signal for displaying clinical information based on the subject-specific information; and displaying clinical information in accordance with the generated display control signal.

Proposed embodiments may thus help a user (such as a medical practitioner, doctor, surgeon, technician, etc.) to be presented with clinical information that is tailored to an individual subject and/or a clinical context. Such information may enable a user to focus on particular clinical information of interest, thus reducing complexity or confusion that may otherwise be caused by presenting additional and/or irrelevant information.

The step of generating a display control signal may be further based on a display position of the clinical information. This can help to ensure that the ordered information is displayed in an optimal manner, for example by accounting for available display space, display rendering constraints, etc.

According to another aspect, there is provided a computer program product for providing or displaying subject-specific information, wherein the computer program product comprises a computer-readable storage medium having computer-readable program code embodied therewith, the computer-readable program code configured to perform all of the steps of a proposed embodiment.

There is also provided a computer system comprising: a computer program product according to proposed embodiment; and one or more processors adapted to perform a method according to a propose concept by execution of the computer-readable program code of said computer program product.

According to another aspect of the invention, there is provided a system for providing subject-specific information. The system comprises: an input interface adapted to obtain a first knowledge model, the first knowledge model comprising a plurality of clinical data elements for a population of subjects and relationship information representing one or more relationships between the clinical data elements, to obtain historical data relating to a sub-population of the population of subjects, the historical data relating to previously-obtained clinical data elements and relationship information for the sub-population, and to obtain subject-specific data relating to a subject, the subject-specific data comprising a plurality of clinical data elements for the subject; and a processing arrangement adapted to generate a second knowledge model based on the first knowledge model and the historical data, and to generate a subject-specific model based on the second knowledge model and the subject-specific data.

In some embodiments, the processing arrangement may be adapted to: based on the historical data relating to a sub-population of the population of subjects, process clinical data elements of the first knowledge model for the sub-population to generate modified clinical data elements for the sub-population; based on the historical data relating to a sub-population of the population of subjects, process relationship information representing one or more relationships between the clinical data elements of the first knowledge model for the sub-population to generate modified relationship information representing one or more relationships between the modified clinical data elements; and generate a second knowledge model comprising: the modified clinical data elements for the sub-population; and the modified relationship information representing one or more relationships between the modified clinical data elements.

According to yet another aspect of the invention, there is provided a system for displaying subject-specific information. The system comprises: a system for providing subject-specific information according to a proposed embodiment; an output interface adapted to generate a display control signal for displaying clinical information based on subject-specific information; and a display unit adapted to display clinical information in accordance with the generated display control signal.

The processing arrangement may be remotely located from the display unit, and the display control signal may thus be communicated to the display unit via a communication link. In this way, a user (such as a medical professional) may have an appropriately arranged display system that can receive and display subject-specific information at a location remotely located from the processing arrangement. Embodiments may therefore enable a user to remotely review ordered subject-specific information using a portable display device, such as a laptop, tablet computer, mobile phone, PDA, etc.

The system may further comprise: a server device comprising the data processing arrangement; and a client device comprising the display unit. Dedicated data processing means may therefore be employed for the purpose of providing subjects-specific information, and generating a display control signal, thus reducing processing requirements or capabilities of other components or devices of the system.

The system may further comprise a client device, wherein the client device comprises the data processing arrangement and the display unit. In other words, a user (such as a doctor or medical professional) may have an appropriately arranged client device (such as a laptop, tablet computer, mobile phone, PDA, etc.) which processes received data in order to provide subject-specific information and generate a control signal.

Thus, it will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is an exemplary flow diagram of a method for providing subject-specific information according to an embodiment;
Figure 2 is an exemplary flow diagram of a method for displaying subject-specific information according to an embodiment;
Figure 3 depicts the result of each stage of a method according to an embodiment;
Figure 4 is a simplified block diagram of a system according to an embodiment; and
Figure 5 is a simplified block diagram of a computer within which one or more parts of an embodiment may be employed.

### DETAILED DESCRIPTION OF THE EMBODIMENTS

Proposed is a concept for enabling the provision of clinical information that is subject-specific in manner which takes account of information relating to a broad/large population of subjects and information relating to previously-obtained data for a subset of the population. Generation of a subject-specific model by combining knowledge from a broad/general model with knowledge regarding a narrower model and the subject may facilitate the provision and/or display of subject-specific clinical information that can help a user to explore and/or analyse the information in a simple and efficient manner.

Accordingly, concepts are proposed which may enrich the provision of clinical information relating to a specific subject by creating a subject-specific model from a general subset of the population model, wherein the general model is refined using historical information relating to a subset of the population used for the general model.

General clinical models may thus be combined with subject-specific historical information in a manner which provides a subject-specific model for analysis or exploration. Clinical information provided by such proposals may thus facilitate simple and intuitive navigation and assessment of subject-specific clinical information with reference to previously-established clinical knowledge. Improved CDS may therefore be provided by proposed embodiments.

Embodiments of the present invention are therefore directed toward enabling the provision of clinical information with reference to subject-specific historical data and general clinical model that has been refined using historical findings so as to facilitate or enhance a CDS process. Further, embodiments may be aimed at enabling the provision of clinical information with simplicity, accuracy and/or clinical context while providing enough detail so that a clinical assessment or decision is facilitated or supported (preferably with increased efficiency and/or reliability).

For a data-driven model, correlation analytics or subject profiling can be performed for single or multiple clinical events (e.g. using prediction models such as logistic regression, correlation analytics, pattern discovery, and/or information-gain based prioritization of features). The combination of clinical events constructed by clinical knowledge and a data-driven model can be implemented by updating and/or extending weights on a graph.

For example, for updating graph weights, correlation (e.g. association) of clinical events from a data-driven model can provide a graph with weights indicating the relationship between events, and they can be used to update graph weights of an initial graph. There may also be events that are not present in the initial graph, such as subsequently occurring (e.g. post-discharge) events. Using graph extension, such new events can be added to the initial graph, and if they have association with existing events, new connections can be defined to link the subsequent (e.g. post-discharge) events to the existing knowledge of the clinical events.

An user or 'expert' (e.g. medical professional, physician) can provide additional information, for example by confirming associations or relationships between events, which can also represented as the personalized perspective of a specific subject. Also, weighting values can be based on a frequency of confirmations for example. Thus, updating and/or extension of a graph can be further applied to a previously updated/extended graph.

By way of example, a potential clinical event can be defined based on clinical data recorded by patients. Based on the categories, it could include (but not be limited to) the following examples:
- Medication related events, e.g. a subject fails to take medication at a prescribed time;
- Vital signs, e.g. a subjects heart rate is larger than 140 but smaller than 160 for 3 days in preceding 5 days
- Health note and symptoms, e.g. a subject recorded a dizzy symptom; and
- Exercise recording, sleeping recording and nutrition recording, e.g. a subject sleeps less than 6 hours in one day.

According to a proposed embodiment, a generic model (e.g. first knowledge model) is initially obtained, which comprises a list of clinical events and their relationships based on existing clinical knowledge and physician's experiences. This model is general/generic for a large population (i.e. not for an individual subject) and thus not updated quickly. Due to the lack of specific clinical knowledge, the weighting values for representing relationships between clinical events can be simplified into several categories, e.g. [0, 0.25, 0.5, 0.75, 1], or simply equalised. Using historical data for a relevant sub-population and the events in the generic model (e.g. data having a relationship weighting which exceeds a threshold value), the generic model can then be updated or refined to generate a new model (e.g. second knowledge model). Thus, this creates a new model with a subset of events of the generic model, but having more accurate relationship weighting (e.g. weighting values that can be real numbers of a continuous range rather than restricted to a small set of available values). Also, extra events (e.g. post-discharge from hospital) can be added to the model as an extension. Finally, a physician's feedback history can be used to further refine the new model (i.e. the second knowledge model). For example, a physicians can provide feedbacks information via a user interface to confirm (or reject) relationships for a specific subject. Combining such physician feedback can thus generates a personalised model for a specific subject. The result may therefore be a subset of events in the model for a sub population (e.g. second knowledge model) with adjusted relationship weights based on subject-specific information from an expert user.

According to proposed concepts, associations between clinical events may be identified, refined and/or analysed for a specific subject based on general clinical knowledge, 'subject-relevant' data and subject-specific data (e.g. physician feedback/input).

Illustrative embodiments may be utilized in many different types of clinical, medical or patient-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

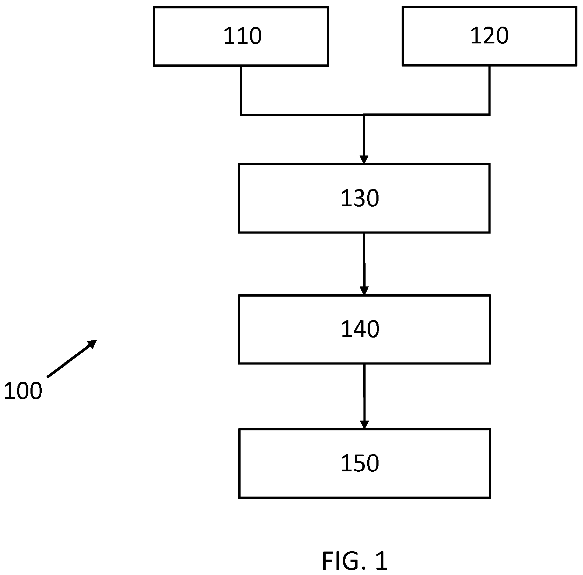

Referring now to Figure 1, there is depicted an exemplary flow diagram of a method 100 method for providing subject-specific information according to an embodiment.

In step 110, a first knowledge model is obtained. The first knowledge model comprises a plurality of clinical data elements for a population of subjects and relationship information representing one or more relationships between the clinical data elements. For instance, for this exemplary embodiment, the obtained first knowledge model comprises clinical knowledge for a population of subjects (e.g. patients in China), and based on existing knowledge, rules, and professional experience. A clinical data element (e.g. obtained from a medical assessment, monitoring instance, medical intervention instance, etc.), for example, can include values of clinical parameters. An example may also include clinical data regarding an abnormality in a specific segment. Also, relationship information representing one or more relationships between the data elements may comprise a weighting value representative of an association between two data elements. Thus, a list of clinical events may therefore be represented as a graph, wherein the weights of the edges are used to indicate different relationship strengths.

The first knowledge model is therefore a generic model representing clinical events and their relationships (e.g. using weighting values).

In step 120, historical data relating to a sub-population of the population of subjects is obtained. Here, the historical data comprises previously-obtained clinical data elements and relationship information representing one or more relationships between the previously-obtained clinical data elements for the sub-population. Here, historical data relating to a sub-population of the population of subjects can provide associated events for the sub-population, and thus enable data analytics for use with the first knowledge model. By way of example, such historical data may relate to a sub-population of patients in Beijing with a disease of interest (e.g. "Disease A").

Based on the first knowledge model and the historical data, a second knowledge model is generated in step 130. In this exemplary embodiment, step 130 of generating a second knowledge model comprises processing, based on the historical data relating to a sub-population of the population of subjects, clinical data elements of the first knowledge model for the sub-population so as to generate modified clinical data elements for the sub-population. Then, based on the historical data relating to a sub-population of the population of subjects, relationship information representing one or more relationships between the clinical data elements of the first knowledge model for the sub-population is processed to generate modified relationship information representing one or more relationships between the modified clinical data elements. A second knowledge model is then generated, wherein the second knowledge model comprises: the modified clinical data elements for the sub-population; and the modified relationship information representing one or more relationships between the modified clinical data elements.

In step 140, subject-specific data relating to a subject of interest is obtained. By way of example, the step 140 of obtaining subject-specific data in this embodiment comprises receiving a user input signal via a user interface, wherein the user input signal comprises a plurality of clinical data elements for the subject. In particular, and by way of example only, the subject-specific data relating to the individual subject of interest comprises feedback history for the subject (e.g. from a physician or medical records), and thus relates to personalized clinical events. In this exemplary embodiment, the step 140 of obtaining subject-specific data relating to a subject comprises obtaining (from a physician or medical professional) historical data comprising previously-generated clinical data elements for the subject.

Based on the second knowledge model and the subject-specific data, a subject-specific model is generated in step 150. In this example, the step 150 of generating a subject-specific model may comprises modifying the second knowledge model based on the subject-specific data so as to generate a subject-specific model.

Here, the subject-specific model comprises a plurality of clinical data elements for the subject and subject-specific relationship information representing relationships between the clinical data elements for the subject. Thus, a personalized model representing associated clinical events for the subject is provided, and this can assists in identification of clinical events and/or relationships unique to the subject.

Here it is noted that, in the embodiment of Figure 1, at least one characteristic value of the subject and the sub-population may be the same. In this way, the second knowledge model may comprise information that is relevant to the subject, because the subject may have one or more characteristics in common with the sub-population. By way of example, the subject may be a member of the sub-population (e.g. the historical data may relate to a sub-population of patients in Beijing with disease A, and the subject may be a patient from Beijing with disease A). This may help to ensure that second knowledge model provides a complementary perspective of clinical events and their relationship(s) that may be relevant to the subject. This may be especially relevant when subject-specific data does not capture more general or broader clinical events and/or relationships.

Accordingly, it will be appreciated that execution of the method of Figure 1 provides subject-specific clinical information, and this may then be leveraged for the purpose of displaying subject-specific information to user (such as a medical professional for example) in a clinical context. Proposed concepts may therefore provide a method for displaying subject-specific information which incorporates an embodiment of providing subject-specific clinical information.

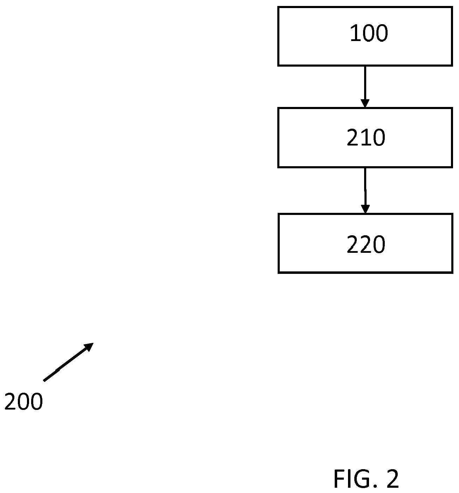

By way of example, Figure 2 depicts an exemplary flow diagram of a method 200 for displaying subject-specific clinical information according to an embodiment, wherein the method 200 employs the method 100 of Figure 1.

More specifically, the method 200 begins with the step 100 of providing subject-specific information according to proposed embodiment of Figure 1. Execution of step 100 therefore results in the provision of a subject-specific model comprising a plurality of clinical data elements for the subject and subject-specific relationship information representing relationships between the clinical data elements for the subject.

In step 210, a display control signal for displaying clinical information is generated based on the subject-specific information. The display control signal is for controlling a display device to display the subject-specific information and may therefore also be generated in consideration of display constraints (such as a display position of the clinical information). In this way, display considerations (such available display space, already-displayed graphical elements, overlapping content, etc.) may be accounted for so as to ensure correct or appropriate display instructions are provided by the control signal.

The clinical information relating to the patient is then displayed in step 220 in accordance with the generated control signal.

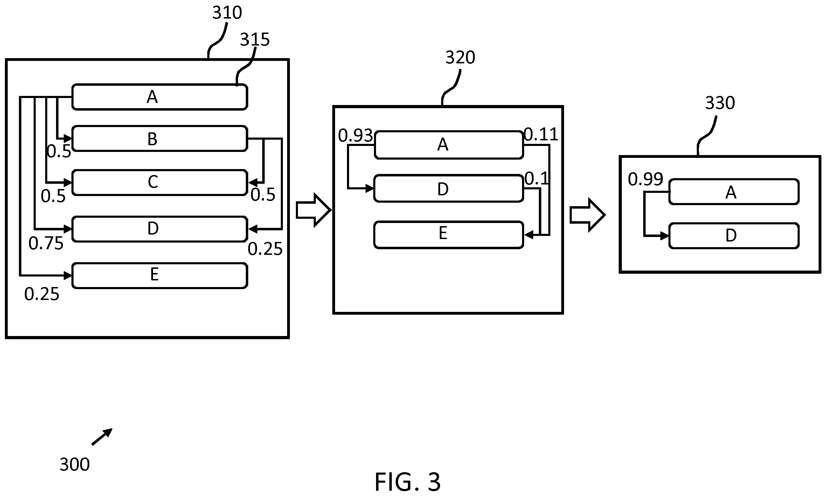

Referring now to Figure 3, an exemplary implementation is depicted wherein the result of each stage is shown. In the first stage, there is provided a first knowledge model 310 comprising a plurality of clinical data elements 315 (e.g. clinical data elements A through E) for a population of subjects and relationship information representing one or more relationships between the clinical data elements. Here, there relationships between the clinical data elements are depicted using arrows and respective weighting values (wherein the weighting values for the first knowledge model can be selected from the set [0, 0.25, 0.5, 0.75, 1]).

In the second stage, there is provided a second knowledge model 320. The second knowledge model 320 comprises a reduced set of clinical data elements 315 (A, D and E) and relationship information representing one or more relationships between the clinical data elements. Here, there relationships between the clinical data elements of the second knowledge model are depicted using arrows and respective weighting values (wherein the weighting values for the second knowledge model are real numbers in the range 0-1).

In the third and final stage, there is provided a subject-specific model 330. The subject-specific model 330 comprises a further reduced set of clinical data elements 315 (A and D) and a relationship between the clinical data elements. Here, there relationship between the clinical data elements of the subject-specific model 330 are depicted using an arrows and respective weighting value (wherein the weighting value comprises a real number in the range 0-1).

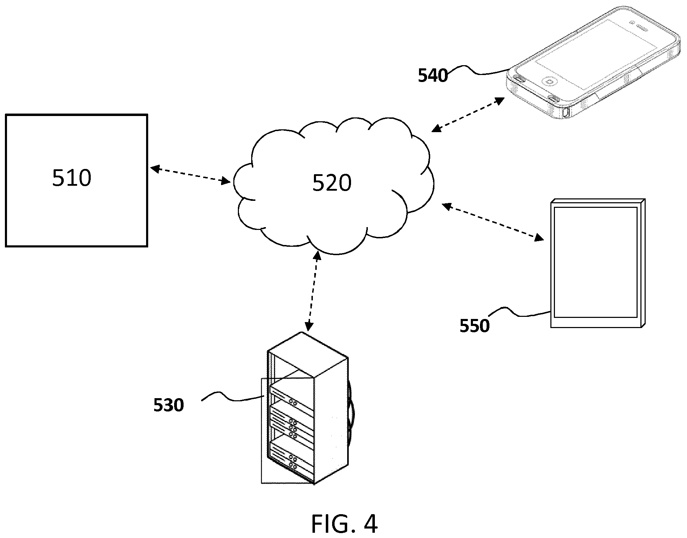

Referring now to Fig. 4, there is depicted an embodiment of a system according to an embodiment of the invention comprising an input system 510 arranged to obtain various sets of information. Here, the input system 510 is adapted to obtain a first knowledge model, the first knowledge model comprising a plurality of clinical data elements for a population of subjects and relationship information representing one or more relationships between the clinical data elements, to obtain historical data relating to a sub-population of the population of subjects, and to obtain subject-specific data relating to a subject. The input system 510 is adapted to output one or more signals which are representative of obtained information/data.

The input system 510 communicates the output signals via the internet 520 (using a wired or wireless connection for example) to a remotely located data processing system 530 (such as server).

The data processing system 530 is adapted to receive the one or more output signals from the input system 510 and process the received signal(s) to generate a subject-specific model. Thus, the data processing system 530 provides a centrally accessible processing resource that can receive information from the input system 510 and run one or more algorithms to order the received information into a subject-specific model. Information relating to the subject-specific model data can be stored by the data processing system (for example, in a database) and provided to other components of the system. Such provision of a subject-specific model may be undertaken in response to a receiving a request (via the internet 520 for example) and/or may be undertaken without request (i.e. 'pushed').

For the purpose of receiving information about a subject from the data processing system 530, and thus to enable subject-specific information to be viewed, the system further comprises first 540 and second 550 mobile computing devices.

Here, the first mobile computing device 540 is a mobile telephone device (such as a smartphone) with a display for displaying subject-specific information in accordance with embodiments of the proposed concepts. The second mobile computing device 550 is a mobile computer such as a Laptop or Tablet computer with a display for displaying subject-specific information in accordance with embodiments of the proposed concepts.

The data processing system 530 is adapted to communicate subject-specific model output signals to the first 540 and second 550 mobile computing devices via the internet 520 (using a wired or wireless connection for example). As mentioned above, this may be undertaken in response to receiving a request from the first 540 or second 550 mobile computing devices.

Based on the received output signals, the first 540 and second 550 mobile computing devices are adapted to display one or more graphical elements in a display area provided by their respective display. For this purpose, the first 540 and second 550 mobile computing devices each comprise a software application for processing, decrypting and/or interpreting received clinical data output signals in order to determine how to display graphical elements. Thus, the first 540 and second 550 mobile computing devices each comprise a processing arrangement adapted to determine an attribute of a clinical map or ordered clinical data, and to generate a display control signal for modifying at least one of the size, shape, position, orientation, pulsation or colour of a graphical element based on the determined attribute of the clinical map and/or ordered clinical data.

The system can therefore communicate information about subject-specific models and/or subject-specific clinical data to users of the first 540 and second 550 mobile computing devices. For example, each of the first 540 and second 550 mobile computing devices may be used to display graphical elements to a medical practitioner, doctor, consultant, technician or caregiver for example.

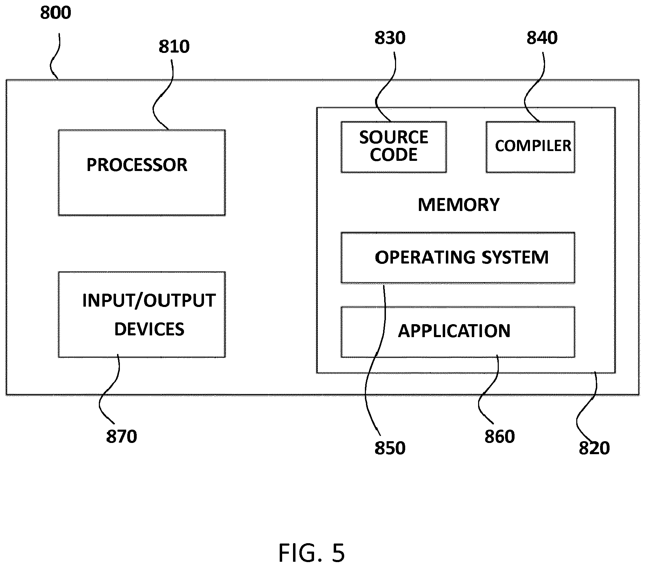

Implementations of the system of Figure 5 may vary between: (i) a situation where the data processing system 530 communicates display-ready subject-specific information, which may for example comprise display data including graphical elements (e.g. in JPEG or other image formats) that are simply displayed to a user of a mobile computing device using conventional image or webpage display (can be web based browser etc.); to (ii) a situation where the data processing system 530 communicates raw data set information that the receiving mobile computing device then transforms to a subject-specific model, processes to determine one or more attributes of the subject-specific model, and then displays graphical elements based on the determined one or more attributes (for example, using local software running on the mobile computing device). Of course, in other implementations, the processing may be shared between the data processing system 530 and a receiving mobile computing device such that part of the subject-specific model generated at data processing system 530 is sent to the mobile computing device for further processing by local dedicated software of the mobile computing device. Embodiments may therefore employ server-side processing, client-side processing, or any combination thereof.

Further, where the data processing system 530 does not 'push' subject-specific information (e.g. subject-specific model output signals), but rather communicates information in response to receiving a request, the user of a device making such a request may be required to confirm or authenticate their identity and/or security credentials in order for information to be communicated.

Figure 5 illustrates an example of a computer 800 within which one or more parts of an embodiment may be employed. Various operations discussed above may utilize the capabilities of the computer 800. For example, one or more parts of a system for providing patient-specific information (or display unit thereof) may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments. The application 860 of the computer 800 may represent various applications, computational units, logic, functional units, processes, operations, virtual entities, and/or modules in accordance with exemplary embodiments, but the application 860 is not meant to be a limitation.

The operating system 850 controls the execution of other computer programs, and provides scheduling, input-output control, file and data management, memory management, and communication control and related services. It is contemplated by the inventors that the application 860 for implementing exemplary embodiments may be applicable on all commercially available operating systems.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850. Furthermore, the application 860 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 870 may include input devices such as, for example but not limited to, a mouse, keyboard, scanner, microphone, camera, etc. Furthermore, the I/O devices 870 may also include output devices, for example but not limited to a printer, display, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 870 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at startup, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 860 and the O/S 850 are read, in whole or in part, by the processor 810, perhaps buffered within the processor 810, and then executed.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A method (100) for providing subject-specific information, the method comprising:
obtaining (110) a first knowledge model (310), the first knowledge model comprising a plurality of clinical data elements for a population of subjects and relationship information representing one or more relationships between the clinical data elements;
obtaining (120) historical data relating to a sub-population of the population of subjects, the historical data relating to previously-obtained clinical data elements and relationship information for the sub-population;
generating (130) a second knowledge model (320) based on the first knowledge model and the historical data;
obtaining (140) subject-specific data relating to a subject, the subject-specific data comprising a plurality of clinical data elements for the subject; and
generating (150) a subject-specific model (330) based on the second knowledge model and the subject-specific data.

2. The method of claim 1, wherein the step (130) of generating a second knowledge model (320) comprises:
based on the historical data relating to a sub-population of the population of subjects, processing clinical data elements of the first knowledge model (310) for the sub-population to generate modified clinical data elements for the sub-population;
based on the historical data relating to a sub-population of the population of subjects, processing relationship information representing one or more relationships between the clinical data elements of the first knowledge model for the sub-population to generate modified relationship information representing one or more relationships between the modified clinical data elements; and
generating a second knowledge model (320) comprising: the modified clinical data elements for the sub-population; and the modified relationship information representing one or more relationships between the modified clinical data elements.

3. The method of any preceding claim, wherein the subject-specific model comprises a plurality of clinical data elements for the subject and subject-specific relationship information representing relationships between the clinical data elements for the subject

4. The method of any preceding claim, wherein the step (150) of generating a subject-specific model comprises:
based on the subject-specific data, modifying the second knowledge model (320) to generate a subject-specific model.

5. The method of any preceding claim, wherein the relationship information representing one or more relationships between the data elements comprises a weighting value representative of an association between two data elements.

6. The method of any preceding claim, wherein at least one characteristic value of the subject and the sub-population is the same.

7. The method of claim 6, wherein the subject is a member of the sub-population.

8. The method of any preceding claim, wherein obtaining (140) subject-specific data relating to a subject comprises:
obtaining historical data comprising previously-generated clinical data elements for the subject.

9. A method (200) for displaying subject-specific information, the method comprising:
providing (100) subject-specific information according to any preceding claim;
generating (210) a display control signal for displaying clinical information based on the subject-specific information; and
displaying (200) clinical information in accordance with the generated display control signal.

10. A computer program product for providing or displaying subj ect-specific information, wherein the computer program product comprises a computer-readable storage medium having computer-readable program code embodied therewith, the computer-readable program code configured to perform all of the steps of any preceding claim.

11. A computer system comprising: a computer program product according to claim 10; and one or more processors adapted to perform a method according to any of claims 1 to 19 by execution of the computer-readable program code of said computer program product.

12. A system for providing subject-specific information, the system comprising:
an input interface (510) adapted to obtain a first knowledge model, the first knowledge model comprising a plurality of clinical data elements for a population of subjects and relationship information representing one or more relationships between the clinical data elements, to obtain historical data relating to a sub-population of the population of subjects, the historical data relating to previously-obtained clinical data elements and relationship information for the sub-population, and to obtain subject-specific data relating to a subject, the subject-specific data comprising a plurality of clinical data elements for the subject; and
a processing arrangement (530) adapted to generate a second knowledge model based on the first knowledge model and the historical data, and to generate a subject-specific model based on the second knowledge model and the subject-specific data.

13. The system of claim 12, wherein the processing arrangement (530) is adapted to:
based on the historical data relating to a sub-population of the population of subjects, process clinical data elements of the first knowledge model for the sub-population to generate modified clinical data elements for the sub-population;
based on the historical data relating to a sub-population of the population of subjects, process relationship information representing one or more relationships between the clinical data elements of the first knowledge model for the sub-population to generate modified relationship information representing one or more relationships between the modified clinical data elements; and
generate a second knowledge model comprising: the modified clinical data elements for the sub-population; and the modified relationship information representing one or more relationships between the modified clinical data elements.

14. The system of claim 12 or 13, wherein the processing arrangement (530) is adapted to:
based on the subject-specific data, modify the second knowledge model to generate a subject-specific model.

15. A system for displaying subject-specific information, the system comprising:
a system for providing subject-specific information according to any of claims 12 to 14;
an output interface adapted to generate a display control signal for displaying clinical information based on the subject-specific information; and
a display unit adapted to display clinical information in accordance with the generated display control signal.
